# EUROPEAN PATENT APPLICATION

(11) **EP 4 660 964 A1**
(43) Date of publication of application: **10.12.2025**
(21) Application number: 25174705.1
(22) Date of filing: 07.05.2025
(51) Int. Cl.: G06V 10/764, G06V 40/10, G06V 40/20

(54) **A COMPUTER-IMPLEMENTED METHOD AND DEVICE FOR INTERACTIVE TRAINING**

(30) Priority: 03.06.2024 HK 32024092195
(71) Applicant: Wu, Zhi Xian, Fo Tan New Territories (HK)
(72) Inventor: Wu, Zhi Xian, Fo Tan New Territories (HK)
(74) Representative: Bewley, Ewan Stuart

(57) **Abstract**

The invention relates to a computer-implemented method and device of interactive training. The method comprises the step of: receiving a video signal comprising a series of images displaying a subject; processing the series of images for detecting actions of the subject, comprising the steps of: extracting from a first image, a first information in relation to a body portion of the subject; locating, automatically, a set of first key points based on the extracted first information from the first image; extracting from the first image, a second information in relation to a hand portion and/or a foot portion of the subject; locating, automatically, a set of second key points based on the extracted second information from the first image; deriving an action characteristic of the subject based on the located sets of first key points and second key points; repeating the processing steps to acquire a series of action characteristics of the subject in respect to time; and determining a first level of action of the subject based on the series of action characteristics.

## Description

### Field of the Invention

The invention relates to the field of interactive training. Particularly but not exclusively, the invention relates to interactive training designed for rehabilitation purposes.

### Background of the Invention

Significant progress has been made in the development of remote monitoring systems to ensure the safety and well-being of patients and elderly individuals. These systems are primarily utilized for monitoring patients, enabling medical professionals and therapists to assess real-time activities and provide guidance for rehabilitation, particularly in the domains of occupational and physical therapy. These therapeutic interventions encompass exercises and activities aimed at aiding patients in recovering from injuries, surgeries, or general physical disabilities associated with aging.

However, the utilization of remote rehabilitation programs is often hindered by the substantial involvement required from therapists, limiting their widespread adoption. Additionally, patients frequently encounter difficulties in independently and remotely completing rehabilitation activities due to the monotonous nature of the exercises and the demanding nature of certain activities, particularly for patients and elderly individuals who may face cognitive or physical challenges when using technical monitoring devices.

In the realm of rehabilitation gaming, commercially available off-the-shelf rehabilitation games generally lack an understanding of the essential role played by therapists. Motor impairments experienced by patients often impede their ability to execute therapeutically appropriate movements, leading them to develop compensatory behaviors that rely on their less affected body parts. Therefore, active involvement of therapists remains crucial in designing therapy sessions, including the selection of suitable exercise games and determination of appropriate difficulty levels, to accommodate the heterogeneous impairment conditions and varying recovery paces of different patients. All these factors render the use of existing remote rehabilitation programs challenging.

### Objects of the Invention

An object of the present invention is to provide a method and a device for interactive training for rehabilitation purposes.

Another object of the present invention is to mitigate or obviate to some degree one or more problems associated with known rehabilitation systems, or at least to provide a useful alternative.

The above objects are met by the combination of features of the main claims; the subclaims disclose further advantageous embodiments of the invention.

One skilled in the art will derive from the following description other objects of the invention. Therefore, the foregoing statements of object are not exhaustive and serve merely to illustrate some of the many objects of the present invention.

### Summary of the Invention

In a first main aspect, the invention provides a computer-implemented method of interactive training. The method comprises receiving a video signal comprising a series of images displaying a subject, the series of images being displayed in conjunction with background scene; processing the series of images for detecting actions of the subject, comprising the steps of extracting from a first image, a first information in relation to a body portion of the subject; locating, automatically, a set of first key points based on the extracted first information from the first image; extracting from the first image, a second information in relation to a hand portion and/or a foot portion of the subject; locating, automatically, a set of second key points based on the extracted second information from the first image; deriving an action characteristic of the subject based on the located sets of first key points and second key points; repeating the processing steps to acquire a series of action characteristics of the subject in respect to time; and determining a first level of action of the subject based on the series of action characteristics.

In a second main aspect, the invention provides a computer-implemented device for interactive training. The device comprises a displaying module configured to display a background scene; an imaging module configured to receive a video signal comprising a series of images displaying a subject in conjunction with the displayed background scene; a processing module configured to detect action of the subject, comprising an extracting module configured to extract from a first image, a first information in relation to a body portion of the subject, and to extract from the first image, a second information in relation to a hand portion and/or a foot portion of the subject; a locating module configured to locate, automatically, a set of first key points based on the extracted first information from the first image, and to locate automatically a set of second key points based on the extracted second information from the first image; a deriving module configured to derive an action characteristic of the subject based on the located sets of first key points and second key points; and an analyzing module configured to acquire, from the processing modules, a series of action characteristics of the subject in respect to time; and subsequently, determine a first level of action of the subject based on the series of action characteristics.

The summary of the invention does not necessarily disclose all the features essential for defining the invention; the invention may reside in a sub-combination of the disclosed features.

### Brief Description of the Drawings

The foregoing and further features of the present invention will be apparent from the following description of preferred embodiments which are provided by way of example only in connection with the accompanying figure, of which:
Fig. 1 is a schematic diagram showing the computer implementing device according to an embodiment of the present invention;
Fig. 2 is a schematic diagram showing an avatar representing the subject with reference to a background scene;
Fig. 3 is a schematic diagram showing the set of 33 first key points representing a body portion of the subject user according to an embodiment of the present invention;
Fig. 4 is a schematic diagram showing additional first key points according to an embodiment of the present invention;
Fig. 5A is a schematic diagram showing the 21 second key points representing a hand portion of the subject user according to an embodiment of the present invention;
Fig. 5B are images showing embodied three-dimensional second information according to an embodiment of the present invention; and
Fig. 6 is a table showing the parameters shown in an embodied report generated by the present invention.

### Description of Preferred Embodiments

The following description is of preferred embodiments by way of example only and without limitation to the combination of features necessary for carrying the invention into effect.

Reference in this specification to "one embodiment" or "an embodiment" means that a particular feature, structure, or characteristic described in connection with the embodiment is included in at least one embodiment of the invention. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments but not other embodiments.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

In the claims hereof, any element expressed as a means for performing a specified function is intended to encompass any way of performing that function including, for example, a) a combination of circuit elements that performs that function or b) software in any form, including, therefore, firmware, microcode or the like, combined with appropriate circuitry for executing that software to perform the function. The invention as defined by such claims resides in the fact that the functionalities provided by the various recited means are combined and brought together in the manner which the claims call for. It is thus regarded that any means that can provide those functionalities are equivalent to those shown herein.

The functions of the various elements or components shown in the figures may be provided through the use of dedicated software, hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor ("DSP") hardware, read-only memory ("ROM") for storing software, random access memory ("RAM"), and non-volatile storage.

The present invention relates to a method and a device for interactive training, including generally physical activities in any exercise settings, such as but are not limited to, home-based training, nursing centers for elderly individuals, general exercise activities within a gym, or rehabilitation training within a physiotherapy or occupational therapy center. Particularly but not exclusively, the invention relates to interactive training designed specifically for rehabilitation purposes for users such as patients recovering from injuries or surgeries, as well as elderly individuals experiencing disability or immobility due to aging. The training activities provided by the present invention can be delivered in a gaming environment, featuring specially designed tasks suitable for rehabilitation of the subject uses. By integrating artificial intelligent (AI) machine learning algorithms with motion detection technology, the present invention enable accurate detection of user's motion within the gaming setting, allowing customization of activity suitability and difficulty levels based on the user's specific physical needs. The incorporated AI technology further facilitates the automatic recommendation of appropriate progressions, while timely assessing and reporting the user's progress to responsible medical professionals or therapists. This information enables the related personnel to prescribe suitable activities or next-level exercises with the appropriate level of demands or difficulties to progress the user's recovery. The present invention is advantageous in that it can be provided in the form of a stand-alone, single device, such as a tablet computer equipped with a camera module for capturing images and/or videos of the user. No additional sensors, wearable monitors, or constant supervision are required for the monitoring, assessing and training process.

Importantly, while the present invention herein described excels in rehabilitation applications, it should not be limited to any specific applications. Instead, the invention is capable of extending to other fields of remote sensing and motion detection in various applications and settings, such as but are not limited to, security surveillance, healthcare monitoring, other IoT (Internet of things) devices, etc.

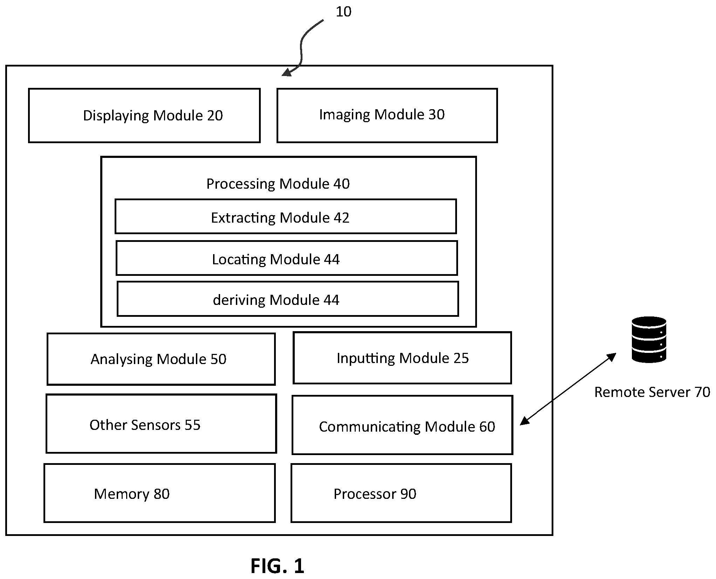

Referring to Fig. 1, shown is a schematic diagram illustrating the technical implementation of the embodied interactive physical training device 10 in accordance with the present invention. In this embodiment, the device 10 is configured as a single, stand-alone, portable computer device, which can take the form of, but is not limited to, a tablet computer, a laptop computer, or a smart phone. The device 10 is equipped with a displaying module 20 capable of displaying and presenting the gaming interface, as well as an imaging module 30 for receiving a video signal comprising a series of frames of images that display the subject user. The received video signal may comprise any video displaying the subject user being captured in real-time. Alternatively, the received video signal may comprise one or more pre-recorded videos showing the subject.

In order to enhance accuracy of the motion detection by the device 10, it is preferred that the face of the user under training is detected and shown in the captured images. More preferably, for optimal detection, the user's body should be positioned such that the entire body is detected and visible in the captured images. In one embodiment, the device 10 preferably comprises an inputting module 25, which may include any known inputting means for the user to manually input responses, instructions, or usage requirements. Alternatively, the inputting module 25 can be integrated with the displaying module 20, allowing for a touchscreen interface to receive user's input, which facilitates a user-friendly instruction input.

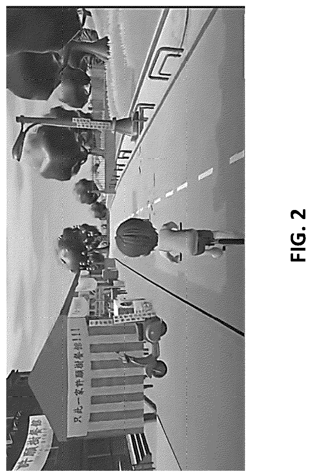

In one embodiment, the captured images can be utilized to generate a customizable avatar representing the user. The avatar is then displayed in conjunction with a background scene, as shown in Fig. 2. The choice of background scene can vary depending on the assigned game tasks for the user. More preferably, the background scene is specifically designed to depict locations, venues or scenery that are familiar to the user to encourage user's engagement. For instance, in the case of a biking exercise, a scenic or local street background, as shown in Fig. 2, could be employed. To enhance personalization, the background scene can further be customized based on various user input such as the user's preferences, personal details such as age, race, gender, home town and/or occupation, as well as geographical settings of the device, etc. The customization further enriches the user's experience during the interactive training sessions. This customization feature is especially useful for patients suffering from dementia and/or memory loss.

In one specific embodiment, the gaming tasks preferably focus on rehabilitation activities which target essential functional aspects of the player, i.e. the subject patient, such as strength, endurance, balance, and flexibility. These activities encompass a wide range of exercises aimed at improving the player's physical capabilities. Example of such activities include, but are not limited to, hand gripping exercises, shoulder flexion movements, horizontal abduction exercises, reciprocal shoulder movements, stepping and side-stepping exercises, cycling simulations, seated knee extensions, hip flexions, sit to stand transitions, mini squats, throwing exercises, and more. These activities are presented in various game settings to provide an engaging and immersive experience for the patients undergoing rehabilitation. For instance, the games may involve tasks like blowing up a balloon, drying a towel, rock climbing, starting a campfire, landing a parachute, putting on clothes, hiking, dancing, pedal boat riding, kicking a bouncing ball, ring tossing, etc. Each of these games is designed with different levels of difficulty, tailored to accommodate players with diverse physical conditions and specific recovery needs. This ensures that the exercises are appropriately challenging and beneficial for users at various stages of their rehabilitation process.

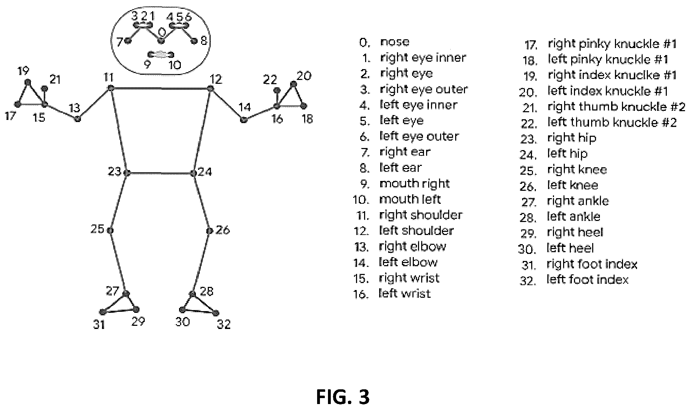

In one embodiment, the device 10 further comprises a processing module 40 configured to detect actions of the subject in response to the game tasks, which are displayed via the displaying module 20. To achieve this, the processing module 40 incorporates an extracting module 42 is configured to extract, from a first image of the series of frames of images comprising the video signal, a first image information in relation to a body portion of the subject. Particularly, the extracted first information preferably comprises two-dimensional image information representing the body portion of the subject. After extraction of the two-dimensional first image information of the body portion, a locating module 44 of the processing module 40 will then automatically locate a set of first body key points, preferably comprising a plurality of first body key points. In one embodiment, the plurality of first body key points may preferably comprise 33 key points correspond to 33 skeletal points of the body portion of the subject, as illustrated in Fig. 3. For different game tasks, different numbers and/or locations of the first key points will be located to facilitate specific pose and motion detection of the subject user.

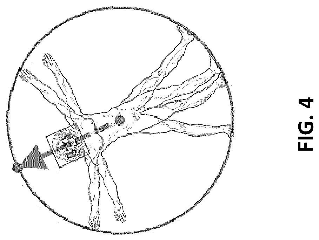

In another embodiment, the set of first key points can be expanded to comprise additional body key points, which serve as points of interest based on virtual alignment of the body. These additional key points may provide useful information for enhanced body tracking and pose detection. For example, the set of first key points may further comprise a mid-point that represents hip of the subject, and a point that defines the radius of a circle circumscribing the subject with the mid-point as the center, as depicted in Fig. 4. In yet a further embodiment, the set of first key points may further comprise a shoulder point. The processing module 40 may then utilize the incline angle defined by the line connecting the shoulder point and the hip, mid-point with the longitudinal axis of the body. This incline angle, along with the 33 first key points, will be processed by the processing device 40 for accurate pose detection of the body. By incorporating these additional key points and pose detection techniques, the present invention allows more comprehensive and precise tracking and analysis of the subject's movements.

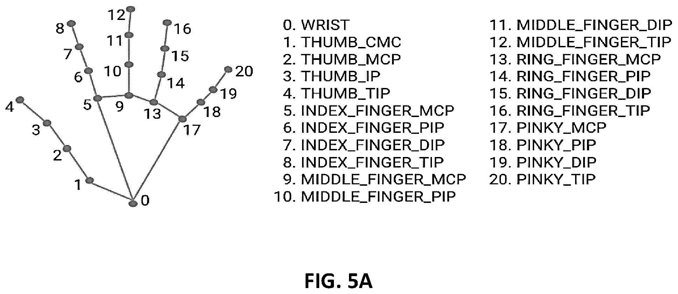

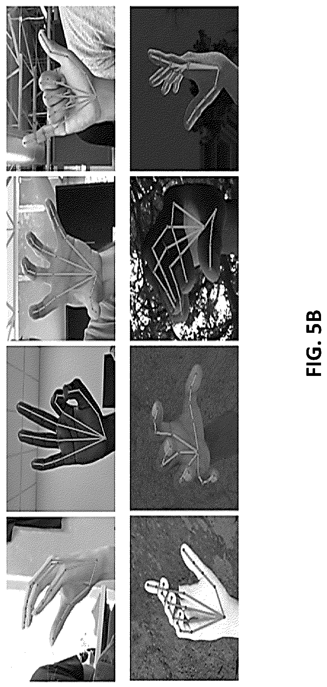

For the next step, the extracting module 42 is further configured to extract, from the same, first image, a second image information in relation to a hand portion and/or a foot portion of the subject. The extracted second information may preferably comprise three-dimensional image information that represents the hand and/or the foot of the subject. Within this second image information, a set of second key points, comprising a plurality of second key points in three-dimensional presentation, is located by the locating module 44. In one embodiment, the plurality of second key points may preferably comprise 21 key points correspond to 21 skeletal points of the hand and/or the foot of the subject, such as the 21 three-dimensional hand-knuckle points as shown in Fig. 5A. For different game tasks, different numbers and/or locations of the second key points will be located to facilitate specific pose and motion detection of the subject user. In one further embodiment, the extracted second information may comprise a cropped image region obtained from the first image, as depicted in Fig. 5B. The cropped regions focus specifically on the hand and/or foot of the subject. Extracting and utilizing these cropped image regions can facilitate more detailed analysis and tracking of the hand and/or foot movements during the interactive physical training process.

The acquired sets of first key points, which represent the body portion of the subject in two-dimension, and the second key points, which represent the hand and/or the foot of the subject in three-dimension based on the first image, are processed using one or more artificial intelligent machine learning algorithms by the deriving module 46 of the processing module 40. These algorithms analyze and extract meaningful information from the key points to derive an action characteristic depicted in the first image. In one optional embodiment, the artificial intelligent machine learning algorithms may employ a heat map regression model for processing by connecting with an infrared sensor connected with the device 10. However, it is important to note that the present invention is not limited to any specific AI models. As long as the processing algorithm does not deviate from the inventive concept of the present invention and achieves the desired technical effect, any known motion detection-based machine learning models can be utilized. These models may include common facial detection, pose detection and/or motion detection algorithms, among others. By leveraging these artificial intelligent machine learning algorithms, the present invention is adapted to accurately interpret the key point data and derive meaningful insights regarding the action characteristics of the subject in the first image. This enables effective analysis and tracking of the subject's movements during the interactive physical training sessions, enhancing the overall training experience.

The processing module 40 performs a repetitive process to identify the sets of first key points and second key points, followed by deriving the action characteristics of the subject in relation to each individual image within the series of images obtained from the video signal. This iterative approach allows for the extraction of a series of action characteristics that capture the subject's movements in respect to time. By analyzing each image in the series, the processing module 40 generates a sequence of action characteristics that provide insights to the subject's actions thought the captured video. The temporal analysis further enables a comprehensive understanding of how the subject's movements evolve during the physical training session. In one embodiment, the iteration process may preferably involve aligning the derived action characteristics of two consecutive images of the series of images. This alignment step enhances the accuracy of the detection process by ensuring a smooth transition and consistency between the action characteristics of adjacent frames. By aligning the derived action characteristics, any discrepancies caused by slight variations or noise in the image sequence can be minimized or eliminated, resulting in more reliable and accurate identification of the subject's actions.

The series of action characteristics obtained from the processing module 40 will then be analyzed by the analyzing module 50 using one or more AI machine learning algorithms. The analysis aims to determine the specific action performed by the subject user based on the derived action characteristics at high accuracy. The determined action represents a first level of action, which serves as a game result indicating the physical condition or performance of the player.

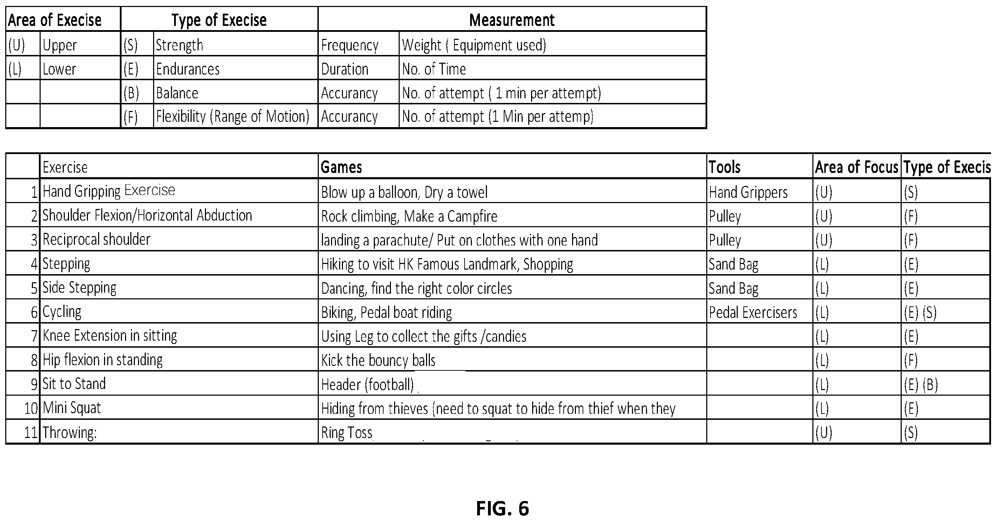

In one embodiment, the obtained first level of action is automatically transmitted through the communication module 60 in the form of a comprehensive report. This report, such as the one illustrated in Fig. 6, is transmitted to the designated users' devices, which may include the administrative platform and the devices of responsible healthcare personnel. By automatically sharing the report, the system ensures efficient and timely dissemination of the user's activity data and performance. The designated users, including healthcare professionals and administrators, can access the report on their devices, enabling them to review the user's progress, identify any concerns, and make informed decisions regarding the individual's training program. Additionally, if immediate attention is deemed necessary, the system generates an instant alert to notify the relevant medical services, prompting them to take appropriate actions. This alert serves as a proactive notification to the relevant medical services, alerting them to the user's condition and prompting immediate action. This feature enhances the system's capability to address emergency situations promptly, ensuring the safety and well-being of the user.

Upon determining the first level of action, the analyzing module 50 is adapted to suggest a second level of action to the subject user. The second level of action may involve game tasks of a different type, varying levels of difficulty, and/or exercises targeting different parts of the user's body, etc. In one embodiment, the analyzing module 50 may automatically suggest the second level of action based on machine learning algorithms trained on existing or past patients' records. By leveraging data gained from analyzing previous users' data, the system can provide personalized suggestions on game types tailored to the individual's needs and abilities.

In another embodiment, the suggestion of the second level of action can be based on one or more predetermined instructions and/or inputted instructions provided by responsible personnels, such as medical professionals or therapists. These instructions take into account the specific medical requirements or goals of the patient, ensuring that the suggested activities align with the user's therapeutic needs. In response to the determined second level of action, the analyzing module 50 may automatically adjust the background scene for the corresponding game tasks. This adjustment can involve modifying the game setting or creating a customized gaming environment specifically designed for the suggested second level of action. By adapting the visual and contextual aspects of the game, the system enhances the user's engagement and immersion, promoting a more effective and enjoyable training experience.

In one preferred embodiment, it is advantageous to integrate one or more of the displaying module 20, imaging module 30, processing module 40, and analyzing module 50 into a single portable device. This consolidation allows for a compact and convenient solution that can be easily carried and used by the subject user. The single device further offers a relatively short detection distance, such as around 1 meter from the subject, enabling the use of the device and thus the rehabilitation training be carried out in a relatively small area of space.

Alternatively, in another preferred embodiment, the displaying module 20, imaging module 30, and processing module 40 can be combined into a single portable device. For the purpose of handling high volume of AI processing required for analysis, the analyzing module 50 may preferably communicate at least part of the acquired data, via the communicating module 60, to a remote server 70 such as a cloud-based server 70, for the data be processed remotely. This distributed architecture leverages the computational power and scalability of cloud computing, enabling robust and efficient processing of the collected data. By offloading the analyzing module 50 to a remote server, the system can handle complex machine learning algorithms and perform computationally intensive operations without overburdening the resources of the portable device. This approach ensures that the analysis is performed swiftly and accurately, providing real-time feedback and suggestions to the subject user. In one further embodiment, the training session of the present invention can be conveniently initiated by scanning a 2-dimensional code, such as a quick-response (QR) code, which facilitates a user-friendly login to the individual user's training program. To begin the session, the patient simply needs to arrange the imaging module 30 to scan their unique QR code, which can be readily displayed on their mobile phone or wearable device, such as a smart watch. Upon detection of the QR code, the present invention is designed to retrieve and display the user's profile, which may include previous activity records showing their progress, weekly schedules showing upcoming activities, and tailored exercise plans recommended by healthcare professionals, etc. This allows the user to track their achievements, stay informed about their training schedule, and adhere to the guidance provided by healthcare professionals for patient compliance. By utilizing the QR code scanning mechanism, the system streamlines the login process, ensuring a hassle-free experience for the user. This method eliminates the need for manual input of login credentials and allows for quick access to the individual's personalized training program.

In summary, the present invention relates to a method and device for interactive physical training, encompassing a wide range of activities in various exercise settings which include, but are not limited to, home-based training, nursing centers for elderly individuals, general exercise activities in gyms or activity rooms, or rehabilitation training in physiotherapy or occupational therapy centers. Specifically, the invention focuses on interactive training designed to aid in the rehabilitation of users such as patients and/or elderly individuals. The training activities offered by the present invention are delivered in a gaming environment, incorporating specially designed tasks tailored for the rehabilitation needs of the users. By combining artificial intelligence (AI) machine learning algorithms with motion detection technology, the present invention enables precise detection of the user's movements in a gaming environment. This allows for the customization of activity suitability and difficulty levels, aligning them with the user's specific physical requirements, and at the same time, encourages users' engagement. Furthermore, the integrated AI technology facilitates automatic recommendations of appropriate progressions, while allowing regularly assessing and reporting the user's progress to responsible medical professionals or therapists. This valuable information empowers these healthcare experts to prescribe suitable activities and determine the appropriate level of challenge or difficulty for advancing the user's recovery. The present invention is preferably provided in the form of a standalone device, such as a tablet computer equipped with a camera module for capturing images and/or videos of the user. This eliminates the need for additional sensors, wearable monitors, or constant supervision during the monitoring, assessment, and training process. By offering a comprehensive and self-contained solution, the present invention enhances the user experience and reduces the burden on both the user and the healthcare professionals involved. This streamlined approach enhances convenience, promotes independent rehabilitation, and minimizes the resources and equipment required for the training process.

While the invention has been illustrated and described in detail in the drawings and foregoing description, the same is to be considered as illustrative and not restrictive in character, it being understood that only exemplary embodiments have been shown and described and do not limit the scope of the invention in any manner. It can be appreciated that any of the features described herein may be used with any embodiment. The illustrative embodiments are not exclusive of each other or of other embodiments not recited herein. Accordingly, the invention also provides embodiments that comprise combinations of one or more of the illustrative embodiments described above. Modifications and variations of the invention as herein set forth can be made without departing from the spirit and scope thereof, and, therefore, only such limitations should be imposed as are indicated by the appended claims.

The present description illustrates the principles of the present invention. It will thus be appreciated that those skilled in the art will be able to devise various arrangements that, although not explicitly described or shown herein, embody the principles of the invention and are included within its spirit and scope.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future, i.e. any elements developed that perform the same function, regardless of structure.

Thus, for example, it will be appreciated by those skilled in the art that the diagrams presented herein represent conceptual views of components embodying the principles of the invention.

It is to be understood that, if any prior art publication is referred to herein, such reference does not constitute an admission that the publication forms a part of the common general knowledge in the art.

## Claims

1. A computer-implemented method of interactive training, comprising:
receiving a video signal comprising a series of images displaying a subject;
processing the series of images for detecting actions of the subject, comprising the steps of:
extracting from a first image, a first information in relation to a body portion of the subject;
locating, automatically, a set of first key points based on the extracted first information from the first image;
extracting from the first image, a second information in relation to a hand portion and/or a foot portion of the subject;
locating, automatically, a set of second key points based on the extracted second information from the first image;
deriving an action characteristic of the subject based on the located sets of first key points and second key points;
repeating the processing steps to acquire a series of action characteristics of the subject in respect to time; and
determining a first level of action of the subject based on the series of action characteristics.

2. The computer-implemented method according to claim 1, wherein, in response to the first level of action determined, suggesting a second level of action to the subject automatically or based on one or more inputted instructions and/or predetermined instructions.

3. The computer-implemented method according to claim 1, wherein the extracted first information comprises two-dimensional information comprising a plurality of first key points representing the body portion of the subject.

4. The computer-implemented method according to claim 3, wherein the plurality of first key points comprise 33 key points correspond to 33 skeletal points of the body portion of the subject.

5. The computer-implemented method according to claim 1, wherein the set of first key points further comprises a mid-point representing hip of the subject, and a point defining a radius of a circle circumscribing the subject with the mid-point.

6. The computer-implemented method according to claim 1, wherein the extracted second information comprises a three-dimensional information comprises a plurality of second key points representing the hand and/or the foot of the subject.

7. The computer-implemented method according to claim 6, wherein the plurality of second key points comprise 21 key points correspond to 21 skeletal points of the hand and/or the foot of the subject.

8. The computer-implemented method according to claim 1, wherein the extracted second information comprises a cropped image region from the first image.

9. The computer-implemented method according to claim 1, wherein the step of repeating the processing steps comprises aligning the derived action characteristics of two consecutive images of the series of images.

10. The computer-implemented method according to claim 2, further comprising displaying an image of the user in conjunction with a background scene; wherein the background scene is automatically adjustable according to the determined first level of action and/or the suggested second level of action.

11. A computer-implemented device for interactive training, comprising:
an imaging module configured to receive a video signal comprising a series of images displaying a subject;
a processing module configured to detect actions of the subject, comprising:
an extracting module configured to extract from a first image, a first information in relation to a body portion of the subject, and to extract from the first image, a second information in relation to a hand portion and/or a foot portion of the subject;
a locating module configured to locate, automatically, a set of first key points based on the extracted first information from the first image, and to locate, automatically, a set of second key points based on the extracted second information from the first image;
a deriving module configured to derive an action characteristic of the subject based on the located sets of first key points and second key points; and
an analysing module configured to acquire, from the processing modules, a series of action characteristics of the subject in respect to time; and subsequently, determine a first level of action of the subject based on the series of action characteristics.

12. The computer-implemented device according to claim 11, wherein the analysing module is further configured to suggest a second level of action to the subject based on the first level of action determined, wherein the analysing module is adapted to automatically suggest the second level of action to the subject, and/or suggestion is based on one or more inputted instructions and/or predetermined instructions.

13. The computer-implemented device according to claim 12, further comprising a displaying module configured to display a background scene in conjunction with an image of the subject; wherein the background scene is automatically adjustable according to the first level of action and/or the suggested second level of action.

14. The computer-implemented device according to claim 11, wherein the first information comprises two-dimensional information comprising a plurality of first key points representing the body portion of the subject; wherein the plurality of first key points comprise 33 key points correspond to 33 skeletal points of the body portion of the subject.

15. The computer-implemented device according to claim 11, wherein the second information comprises three-dimensional information comprising a plurality of second key points representing a hand portion and/or a foot portion of the subject; wherein the plurality of second key points comprise 21 key points correspond to 21 skeletal points of the hand and/or the foot of the subject.

## Amended claims

### Amended claims in accordance with Rule 137(2) EPC.

1. A computer-implemented method of interactive training, comprising:
receiving a video signal comprising a series of images displaying a subject;
processing the series of images for detecting actions of the subject, comprising the steps of:
extracting from a first image, a first information in relation to a body portion of the subject;
locating, automatically, a set of first key points based on the extracted first information from the first image;
extracting from the first image, a second information in relation to a hand portion and/or a foot portion of the subject; wherein the extracted second information comprises a cropped image region from the first image;
locating, automatically, a set of second key points based on the extracted second information from the first image;
deriving an action characteristic of the subject based on the located sets of first key points and second key points;
repeating the processing steps to acquire a series of action characteristics of the subject in respect to time; and
determining a first level of action of the subject based on the series of action characteristics.

2. The computer-implemented method according to claim **1,** wherein, in response to the first level of action determined, suggesting a second level of action to the subject automatically or based on one or more inputted instructions and/or predetermined instructions.

3. The computer-implemented method according to claim 1, wherein the extracted first information comprises two-dimensional information comprising a plurality of first key points representing the body portion of the subject.

4. The computer-implemented method according to claim 3, wherein the plurality of first key points comprise 33 key points correspond to 33 skeletal points of the body portion of the subject.

5. The computer-implemented method according to claim 1, wherein the set of first key points further comprises a mid-point representing hip of the subject, and a point defining a radius of a circle circumscribing the subject with the mid-point.

6. The computer-implemented method according to claim 1, wherein the extracted second information comprises a three-dimensional information comprises a plurality of second key points representing the hand and/or the foot of the subject.

7. The computer-implemented method according to claim 6, wherein the plurality of second key points comprise 21 key points correspond to 21 skeletal points of the hand and/or the foot of the subject.

8. The computer-implemented method according to claim 1, wherein the step of repeating the processing steps comprises aligning the derived action characteristics of two consecutive images of the series of images.

9. The computer-implemented method according to claim 2, further comprising displaying an image of the user in conjunction with a background scene; wherein the background scene is automatically adjustable according to the determined first level of action and/or the suggested second level of action.

10. A computer-implemented device (10) for interactive training, comprising:
an imaging module (30) configured to receive a video signal comprising a series of images displaying a subject;
a processing module (40) configured to detect actions of the subject, comprising:
an extracting module (42) configured to extract from a first image, a first information in relation to a body portion of the subject, and to extract from the first image, a second information in relation to a hand portion and/or a foot portion of the subject; wherein the extracted second information comprises a cropped image region from the first image;
a locating module (44) configured to locate, automatically, a set of first key points based on the extracted first information from the first image, and to locate, automatically, a set of second key points based on the extracted second information from the first image;
a deriving module (44) configured to derive an action characteristic of the subject based on the located sets of first key points and second key points; and
an analysing module (50) configured to acquire, from the processing modules (40), a series of action characteristics of the subject in respect to time; and subsequently, determine a first level of action of the subject based on the series of action characteristics.

11. The computer-implemented device (10) according to claim 10, wherein the analysing module (50) is further configured to suggest a second level of action to the subject based on the first level of action determined, wherein the analysing module (50) is adapted to automatically suggest the second level of action to the subject, and/or suggestion is based on one or more inputted instructions and/or predetermined instructions.

12. The computer-implemented device (10) according to claim 11, further comprising a displaying module (20) configured to display a background scene in conjunction with an image of the subject; wherein the background scene is automatically adjustable according to the first level of action and/or the suggested second level of action.

13. The computer-implemented device (10) according to claim 10, wherein the first information comprises two-dimensional information comprising a plurality of first key points representing the body portion of the subject; wherein the plurality of first key points comprise 33 key points correspond to 33 skeletal points of the body portion of the subject.

14. The computer-implemented device (10) according to claim 10, wherein the second information comprises three-dimensional information comprising a plurality of second key points representing a hand portion and/or a foot portion of the subject; wherein the plurality of second key points comprise 21 key points correspond to 21 skeletal points of the hand and/or the foot of the subject.
